(19) Europäisches Patentamt
European Patent Office
Office européen des brevets

(11) EP 2 483 672 B1

## (12) EUROPEAN PATENT SPECIFICATION

(45) Date of publication and mention
of the grant of the patent:
**13.09.2017 Bulletin 2017/37**

(21) Application number: **10819958.9**

(22) Date of filing: **29.09.2010**

(51) Int Cl.:
*G01N 27/06* (2006.01)     *G01N 27/07* (2006.01)
*G01R 27/22* (2006.01)     *G01V 3/06* (2006.01)

(86) International application number:
**PCT/FI2010/050749**

(87) International publication number:
**WO 2011/039416 (07.04.2011 Gazette 2011/14)**

### (54) THREE DIMENSIONAL IMAGING OF A MASS FLOW

DREIDIMENSIONALE ABBILDUNG EINES MASSENFLUSSES

RÉALISATION D'IMAGE EN TROIS DIMENSIONS D'UN ÉCOULEMENT MASSIQUE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **29.09.2009 FI 20095994**

(43) Date of publication of application:
**08.08.2012 Bulletin 2012/32**

(73) Proprietor: **OUTOTEC (FINLAND) OY**
**02230 Espoo (FI)**

(72) Inventors:
• **KAIPIO, Jari**
**Murrays Bay, North Shore 0630 (NZ)**
• **LEHIKOINEN, Anssi**
**70820 Kuopio (FI)**
• **VOUTILAINEN, Arto**
**FI-70820 Kuopio (FI)**
• **VAUHKONEN, Marko**
**FI-70100 Kuopio (FI)**

(74) Representative: **Papula Oy**
**P.O. Box 981**
**00101 Helsinki (FI)**

(56) References cited:
**EP-A1- 1 411 348        WO-A1-02/053029**
**WO-A1-2008/011716       JP-A- 2007 327 950**
**US-A1- 2005 156 604**

• **VOUTILAINEN A ET AL: "Three-dimensional nonstationary electrical impedance tomography with a single electrode layer", MEASUREMENT SCIENCE AND TECHNOLOGY, IOP, BRISTOL, GB, vol. 21, no. 3, 1 March 2010 (2010-03-01), page 35107, XP020174446, ISSN: 0957-0233**
• **A. SEPPÄNEN ET AL: "State estimation in process tomography-Three-dimensional impedance imaging of moving fluids", INTERNATIONAL JOURNAL OF NUMERICAL METHODS IN ENGINEERING., vol. 73, no. 11, 12 March 2008 (2008-03-12), pages 1651-1670, XP055235916, GB ISSN: 0029-5981, DOI: 10.1002/nme.2142**
• **SEPPXNEN A. ET AL: 'An experimental evaluation of state estimation with fluid dynamical models in process tomography' CHEMICAL ENGINEERING JOURNAL vol. 127, 2007, pages 23 - 30, XP005903392**

**Description**

FIELD OF THE INVENTION

The present invention relates to imaging a target volume by means of impedance tomography. Particularly, the present invention focuses on determining the electrical conductivity distribution of a mass flow in a three dimensional target volume.

BACKGROUND OF THE INVENTION

There are numerous situations in different kinds of processes in e.g. mining, food processing or pulp and paper industries wherein there is a need to investigate the internal properties of a mass flow in a pipeline or some container. The object of the investigation can be e.g. the number and sort of gas bubbles in a liquid or the mixing of an additional substance supplied into the flow.

One useful technique for said investigation of the properties of the flow is impedance tomography or impedance spectroscopy tomography. The word "tomography" usually refers to cross-sectional imaging. By impedance tomography is meant in general electrical measurements made by means of electrodes placed on the surface of or within the target, and determination of the target's electrical conductivity distribution based on the measurements. Areal variations in the conductivity determined as a result of the impedance tomography indicate variations in the quality of the flowing mass and can thus give information e.g. about gas bubbles or other non-uniformities of the mass. In typical measurements, current or voltage is supplied between two particular electrodes and the voltage or the current, correspondingly, is measured between these or some other pair(s) of electrodes. Naturally, several pairs of supplying as well as measuring electrodes can be used simultaneously. By impedance tomography, as its basic form, is usually meant measurements carried out at one single frequency. When impedance measurements in general are performed at several frequencies over a specified frequency range, the usually used term is impedance spectroscopy. The present invention relates to technology where the aim is to produce reconstructions, i.e. tomography images over a frequency range. This technology is often called Electrical Impedance Spectroscopy Tomography EIST. In this document, the expression "impedance tomography" is used to cover both impedance tomography in its conventional meaning and EIST.

As stated above, in impedance tomography an estimate of the electrical conductivity of the target as a function of location is calculated on the basis of measurement results. Thus, the problem in question is an inverse problem where the measured observations, i.e. the voltage or the current, are used to determine the actual situation, i.e. the conductivity distribution which caused the observations. The calculation is based on a mathematical model determining the relations between the injected currents (or voltages), the electrical conductivity distribution of the target, and the voltages (or currents) on the electrodes. The voltages and currents according to the model are compared with the supplied and the measured ones, and the differences between them are minimized by adjusting the parameters of the model until a desired accuracy is achieved.

A conventional sensor configuration used in determining the properties of a mass flow in a pipeline comprises electrodes placed symmetrically in an electrode ring on the inner surface of the pipe. In this conventional configuration, the electrode ring lies in a plane perpendicular to the flow direction. This kind of sensor arrangement enables forming approximate, two-dimensional section images of the mass flow.

For producing a three-dimensional image corresponding to the conductivity distribution of the mass flow in a three dimensional target volume, arrangements comprising several sequential electrode rings along the pipeline are known. Of course, also in the case of electrodes placed within one plane, the voltage and current distributions extend to some extent also outside this plane, but e.g. an air bubble outside the plane causes in the voltage and the current fields a deviation which is independent on the actual side of the plane on which the bubble lies. Thus, the measurement does not produce information about on which side of the electrode plane the bubble actually is. When also the direction along the flow direction is included in the measurement geometry, the measurements give information also about the conductivity distribution in this direction. However, several electrode rings make the measurement sensor rather complex and increase its size. A larger amount of electrodes naturally also increases the cost of the sensor.

Also, solutions are known where, in addition to the stationary situations, the conductivity distribution is determined as a function of time. In this kind of case, reconstructing the conductivity distribution based on a model and measurements is a question of dynamical inversion. A time-varying conductivity distribution thus generated can be used to investigate e.g. drifting of air bubbles or some additional material within the flow. In dynamical inversion, also the motion of the mass flow is included in the conductivity model forming the basis for the calculation. The motion of the mass flow in the pipeline can be modelled e.g. by means of a suitable flow model combined with a convection-diffusion model. The dynamics involved in the mass flow can also be described in a more straightforward manner by some simple time series analysis model.

The above-described sensor configurations comprising several sequential electrode rings are utilized also in the three dimensional dynamic inversion cases of the prior art solutions. The calculation is based on a state space model of the conductivity, the state to be examined being the electrical conductivity in the target volume. The calculation method is the so called state estimation, the principles of which are well known e.g. in the field of automatic control engineering. Instead of determining just one stationary state, in the state estimation technique also the following state is estimated on the basis of the previous state(s). In addition to an observation model comprising, in the case of impedance tomography, the voltage/current/conductivity distribution, the state space model also comprises a so called evolution model describing how the electrical conductivity changes as a function of time.

Like in inverse problems in general, also in state estimation the situations according to the observation and the evolution models are compared with the supplied and the measured situations, and the state space model is adjusted as needed to minimize the differences between the modelled results and the real, i.e. the supplied and the measured values. Several alternative practical level methods are known to carry out the calculations. Examples of solutions exploiting the dynamic inversion state estimation are described in more detail e.g. in the references [1] - [4] listed below:

[1] A. Seppänen, M. Vauhkonen, P.J. Vauhkonen, E. Somersalo, J.P. Kaipio: "State estimation with fluid dynamical evolution models in process tomography -- An application with impedance tomography", Inverse Problems 17:467-483, 2001.

[2] A. Seppänen, M. Vauhkonen, P.J. Vauhkonen, E. Somersalo, J.P. Kaipio: "Fluid dynamical models and state estimation in process tomography: Effect due to inaccuracies in flow fields", J. Electr. Imag., 10(3); 630-640, 2001.

[3] A. Seppänen, L. Heikkinen, T. Savolainen, A. Voutilainen, E. Somersalo, J.P. Kaipio : "An experimental evaluation of state estimation with fluid dynamical models in process tomography", Chemical Engineering Journal, 127: 23-30, 2007

[4] A. Seppänen, M. Vauhkonen, P.J. Vauhkonen, A. Voutilainen, J.P. Kaipio: "State estimation in three dimensional impedance imaging - Use of fluid dynamical evolution models", International Journal for Numerical Methods in Engineering, 73: 1651-1670, 2008

SUMMARY OF THE INVENTION

The method, apparatus, and computer program according to the present invention are characterized by what is presented in claim 1, 7, and 13, correspondingly.

The method of the present invention is a method for determining the electrical conductivity of a mass flow in a three dimensional target volume, i.e. the three dimensional conductivity distribution within said volume. The mass flow can consist of any liquid material possibly containing also solid and/or gaseous substances. The term "flow" has to be understood widely here. In the most typical applications the mass continuously flows through a target volume within a pipeline. However, the target volume can also lie in a container when it is also conceivable that no actual flow-through exists but the flow may comprise e.g. circular motion around the container.

Examples of the applications of the present invention are different kinds of processes in e.g. mining, food processing or pulp and paper industries.

The method comprises the steps of: collecting current or voltage values generated by supplying alternating voltage or alternating current to the target volume and measuring the current or the voltage, correspondingly, thereby induced in the target volume; providing a state space model which defines the relationships between the electrical conductivity, the voltage and the current in the target volume and which also defines the evolution of the electrical conductivity as a function of time; comparing the currents and/or the voltages according to the state space model with the supplied and the measured ones; modifying as needed the state space model to decrease the differences between the calculated and the measured results until a predetermined consistency between the model and the measured values is achieved; and determining the electrical conductivity of the mass flow in the target volume according to the modified state space model.

The step of collecting the current or voltage values can be performed using principles and equipment as such known in the art. For example, when the measured values are presented in electronic form, suitable data transfer and storage means can be used. Said means can comprise e.g. a computer being connected to electrical measurement equipment.

The basic principle of the mathematical state space model of a type utilized in the present invention can be determined e.g. by the following equations:

$$V_t = U_t(\sigma_t) + v_t$$

$$\sigma_{t+1} = f_t(\sigma_t) + w_t,$$

where the upper equation refers to an observation model and the lower one to an evolution model. $V_t$ denotes observation(s), e.g. the measured voltages, at a time $t$, $U_t(\sigma_t)$ determines a mathematical model based on which the corresponding voltages can be calculated, $\sigma_t$ is the electrical conductivity distribution at a time $t$, $v_t$ is the so called observation noise, $f_t(\sigma_t)$ determines an evolution model, and $w_t$ the noise therein. The actual state space model can be any suitable one of the state space model types known in the field. In general, the goal in the state estimation technique is to find estimates for the unknown state variables $\sigma_t$ for $t$ = 1,2,...,n. The state estimation approach leads to a recursive computational algorithm. The most common algorithms used in state estimation methods in general are the Kalman filter and its variants such as the Kalman predictor, the Kalman smoother, the fixed-lag smoother, the extended Kalman filter, the iterated extended Kalman filter, and so on. These are applicable in the case of linear state space models with Gaussian noise processes. In other types of more complicated cases the state variables, i.e. in this case the electrical conductivity, can be estimated using e.g. particle filters.

For said comparing of the currents and voltages according to the state space model with the supplied and the measured ones, the excitation signal in the model is first set consistent with the truly supplied one. Then the response voltage or current values, at the locations corresponding the conditions used in supplying and measuring the voltage and current signals, according to the state space model are calculated and the calculated values are compared with those values actually measured. Said iteration by modifying the state space model in order to decrease the differences between the calculated and the supplied and measured results is continued until the desired consistency between the model and the measured values is achieved. After the iterative modification, the time-varying electrical conductivity distribution of the mass flow in the three-dimensional target volume is determined according to the modified state space model. This determination is based on the equations involved in the state space model. By using those equations, the conductivity distribution can be presented, for example, as a data set comprising calculated conductivity values for discrete points within the three dimensional target volume.

Preferably, at least a part of the method according to the present invention is performed automatically by means of a computing software, i.e. a computer program, installed e.g. in a production control system of the industrial plant at issue. The results are then quickly available in electronic form for further analysis and process control purposes. By means of such software, one or more of the steps of the method can also be operated at least partially manually.

The present invention is based on a surprising observation by the inventors that when using dynamical inversion and state estimation in the electrical conductivity determination, it is possible to determine the time-dependent state of the electrical conductivity of the mass flow in a three dimensional target volume by means of conductivity information originating from one single plane only. Thus, according to the core principles of the present invention, the collected current or voltage values are selected to consist of results of measurements performed substantially within one plane, and the state space model is provided so as to comprise the time-dependent flow field of the mass flow within the target volume. Thus, in contrast to the prior art three dimensional conductivity determination methods utilizing measured information gathered three dimensionally along the direction of the mass flow, in the present invention the measurements can be performed within one single plane only. This is a very advantageous development step and opens a great variety of novel and enhanced possibilities for mass flow imaging. From a determination method point of view, the present invention highly increases the efficiency of the conductivity determination as a three dimensional analysis can be now performed on two-dimensional raw data. In other words, much more information can now be extracted from two dimensional measurement data than is possible with the prior art solutions. From the measurement equipment point of view, the single-plane measurement approach enables radically smaller sensor heads which can be used in process equipment locations which were not possible with the traditional sensors. On the other hand, due to the smaller size and simpler mechanical structure of the sensor head enabled by the present invention, also the manufacturing costs remain lower. In addition, also the electronics and the software needed to supply and measure the electrical signals can be simpler.

The key feature enabling the three dimensional conductivity determination on the basis of the current or voltage values from one single plane only is said state space model comprising the time-dependent flow field of the mass flow. The expression "comprising the time-dependent flow field of the mass flow" means here that the state space model, actually the evolution model equations, contains information needed so that also the time-varying directions and velocities of the mass flow in different points of the target volume could be taken into account in the calculations. This removes the symmetry-related identifying problem inherently present in two dimensional measurements, i.e. the problem arising from the fact that two different situations which are symmetrical with respect to the measurement plane produce identical measurement results. Thus, to gather sufficient observations for the three dimensional conductivity determination, it is sufficient in the present invention to ensure that the measurement configuration is capable of supplying and measuring currents and voltages within said measurement plane.

To summarize, the present invention uses a model for the unknown parameter, wherein this parameter, i.e. the conductivity, is represented as a time-dependent process. The model is formed so as to remove the symmetry-related ambiguity problem arising from only one single observation/measurement plane within the three dimensional target volume. Time-dependent models as such are known also in the prior art. However, in the known solutions either 1) the measurement configuration has been three dimensional, thus not causing the symmetry-related ambiguity problem at all; 2) a *time*-independent model has been used for the unknown parameter; or 3) the unknown parameter has been assumed to be symmetrical (e.g. a so called two-and-half-dimensional model). Thus, the present invention uses for the first time the time-dependent model itself to eliminate the ambiguity problem.

The evolution model as part of the state space model plays a crucial role in the computations according to the present invention, thus the proper selection of the evolution model type is very important. For example, the commonly used random walk model cannot be used. As already stated above, the symmetry-related unidentifiability problem has to be solved by said inclusion of the time-dependent flow field of the mass flow in the state space model.

One preferable choice is to determine the time variation of the conductivity by a convection-diffusion equation. From the convection-diffusion model, a differential equation group is obtained through FEM (Finite Element Method) discretization to describe the conductivity changes in the nodal points of the calculation area. Use of said model requires that the conductivities of the nodal points at the inflow edge of the calculation area are known. However, since the conductivity distribution as a whole is to be estimated and is not known at any point of the target volume, the conductivity at the inflow edge has to be described by some mathematical model. The possible models are numerous, but they may vary in their effectiveness. One example of a suitable approach is described in the following.

In the example, the conductivity distribution at the inflow edge is presented by means of two components, first of them modeling the fluctuation of the conductivity, and the second one the local inhomogeneities. The first component is locally constant at the inflow edge (homogeneous, i.e. no spatial variation), but it is modeled as a time-dependent parameter (process). The time evolution of this term is described by a higher order (>1) time series model or by a stochastic differential equation. In this example, an autoregressive AR(2) model is used. The second component represents the inhomogeneities deviating from the average value at the inflow edge. Also the time evolution of this component (a vector valued process) is described by a higher order time series model or by a stochastic differential equation. In this example, a second order AR(2) model is used also for this component. The conductivity distribution at the inflow edge is thus modeled as the sum of these two components. In this example, the model coefficients of the homogeneous part are chosen so that the predicted conductivity at a specific time is linearly extrapolated from the two previous values. The variance of the noise term related to the model is selected on the basis of the expected rate of variations in the average conductivity of the target volume. The model at issue is unstable, i.e. the variance grows unlimitedly along time. The model is nevertheless useful because it ensures a time-wise smooth behavior and does not limit the conductivity values but allows large fluctuations thereof. Since the observations provide information on the average conductivity in the surroundings of the measurement plane, the estimation of the homogeneous component of the model is stabilized and it (the homogeneous component) does not range arbitrarily during the estimation. The coefficients of the inhomogeneous component and the covariance are selected in this example so that the process is time-wise smooth/correlated, the average is zero, and the structure of the covariance corresponds to both the expected fluctuations and the rate of change. The covariance of the noise term can be rightly chosen so that the noise is spatially correlated. This can be accomplished, for example, by constructing a smooth two-dimensional process model across the inflow boundary.

The example under discussion here is based on an assumption that the equipment used to collect the current or voltage values is coupled to the target volume via a plurality of separate electrodes. If also the contact impedances of the electrodes are quantities to be estimated, a similar evolution model needs to be determined also for them. The time evolution of each of the contact impedances to be estimated is described by a higher order time series model or a stochastic differential equation. The variance of the noise term is selected according to the expected behavior of the rate of change of the contact impedances. If some of the electrodes are not used to supply the excitation signals, it is useful to set the variance of the noise of the terms describing the contact impedances of these electrodes very low. At a practical level, there are several alternative approaches for estimating the contact impedances. One possibility is to estimate the supplying electrodes separately and set for the contact impedances of the rest of the electrodes a (time-dependent) value which corresponds to the average of the estimated contact impedances.

The model needed in the actual estimation procedure is formed by collecting all the unknown terms in the models described above in a same state vector and by generating for it a single common model on the basis of the separate models. Hence, depending on the physical model describing the actual situation, a linear or non-linear time-dependent computational model is achieved, which model can be used as a state evolution model in the computation algorithm (e.g. a Kalman filter, EKF, IEKF).

In a preferred embodiment of the present invention, for the steps of comparing the currents and/or voltages according to the state space model with the supplied and the measured ones and modifying as needed the state space model, a data set comprising current and/or voltage values according to the state space model, correspondingly, within the target volume is generated. This kind of data set can be stored and processed, e.g. updated after every modification round of the state space model, efficiently and automatically by means of an electrical data processing device and suitable software run therein.

In one embodiment of the present invention, the step of collecting current or voltage values comprises, using a plurality of electrodes in a measuring connection with the target volume, the electrodes being placed substantially within one plane, supplying alternating voltage or alternating current to the target volume between two of the electrodes, and measuring the current or the voltage, correspondingly, between two of the electrodes. A plurality of electrodes this way placed and measurements performed by means of these electrodes is an efficient way to carry out the raw data collection.

The electrodes can be of any known type and structure suitable for impedance tomography measurements and the details or the number thereof are not in the core of the invention. The measuring connection means that each of the electrodes is able to supply an excitation signal to and/or measure a response signal from the mass flow. Thus, preferably but not necessarily, the electrodes are in direct contact with the mass flow. The electrodes can be located, for example, on the wall(s) of or within the inner volume of a pipeline or other structure carrying the mass flow to be investigated. Either voltage or current can be utilized as the excitation signal in the measurements used to investigate the conductivity. In the former case the parameter to be measured is most typically current and in the latter one voltage. However, it is also possible that the measured parameter is the same as the supplied one. In this case, i.e. if both the supplied and the measured parameter is voltage or both the supplied and the measured parameter is current, at least one of the two measuring electrodes has to be different from the two supplying electrodes. Said expression of supplying voltage or current between two of the electrodes means of course that the excitation signal is supplied *at least* between two of the electrodes. Naturally, it is possible and often also reasonable to supply e.g. excitation current simultaneously between several pairs of electrodes. In principle, it is even possible to supply simultaneously current between some pair of electrodes and voltage between another pair of electrodes. Similarly, the measurements can of course be performed simultaneously between several pairs of electrodes, and the measurements can be continued longer than the excitation signal is supplied and/or repeated several times per each excitation signal supply.

Preferably, the electrodes according to the present invention are placed so as to set the plane which they determine substantially perpendicular with respect to the average propagation direction of the mass flow in the target volume. The perpendicular position minimizes the space needed by the sensor comprising the electrodes, thus enabling very compact sensor configuration.

The electrodes can be attached, for example, to a longitudinal measuring probe arranged to extend to the target volume. In a very preferred embodiment of the present invention, the electrodes are placed in an annular configuration surrounding the target volume. In this embodiment, very accurate measurements can be achieved throughout the entire target volume cross section enclosed by the electrode ring.

The apparatus of the present invention is an apparatus for determining electrical conductivity of a mass flow in a three dimensional target volume. The apparatus comprises: collecting means for collecting current or voltage values generated by supplying alternating voltage or alternating current to the target volume and measuring the current or the voltage, correspondingly, thereby induced in the target volume; first determining means for determining a state space model which defines the relationships between the electrical conductivity, the voltage and the current in the target volume and which also defines the evolution of the electrical conductivity as a function of time; comparing means for comparing the currents and/or the voltages according to the state space model with the supplied and the measured ones; modifying means for modifying as needed the state space model to decrease the differences between the calculated and the measured results; and second determining means for determining the electrical conductivity of the mass flow in the target volume according to the modified state space model.

The collecting means can be implemented as any type of one or more devices suitable for collecting said current or voltage values. As an example, the collecting means can comprise a computer arranged to receive, via some suitable data transfer connection, measured data from an external measurement device.

Similarly, the first and second determining means, comparing means, and modifying means can comprise equipment of any type known in the art and capable of performing the intended operations thereof. In practice, at least some of the first and second determining, comparing and modifying operations are most preferably performed by means of computer program(s) executing said operations at least partially automatically. As is clear for a person skilled in the art, one or more of said intended operations can be actually performed by a same single device, e.g. a computer and one or more suitable software run therein. In other words, said separately defined means does not necessarily mean here separate actual devices.

As the core of the invention, the collecting means are arranged to select the collected current or voltage values so as to consist of results of measurements performed substantially within one plane; and the first determining means are arranged to determine the state space model so as to comprise the time-dependent flow field of the mass flow within the target volume. The principles of as well as the advantages provided by this basic idea of the present invention are already discussed above in the context of the method aspect of the present invention. The same applies to the preferred embodiments below.

Preferably, the first determining means are arranged to determine the evolution of the electrical conductivity as a function of time in the state space model by means of a convection-diffusion model.

In a preferred embodiment, for comparing the currents and/or voltages according to the state space model with the supplied and the measured ones and modifying as needed the state space model, the apparatus comprises means for generating a data set comprising current and/or voltage values according to the state space model, correspondingly, within the target volume.

In one preferred embodiment, the collecting means comprise electrodes in a measuring connection with the target volume, the electrodes being placed substantially within one plane, and supplying and measuring means for supplying alternating voltage or alternating current to the target volume between two of the electrodes and measuring the current or the voltage between two of the electrodes. As already stated above in the section concerning the method, the electrodes can be of any known type suitable for supplying and measuring voltage and/or current signals. The supplying and the measuring electrodes can be the same, or different groups of electrodes can be used for supplying and measuring the signals.

Said supplying and measuring means can comprise any combination of known electrical and electronics devices, possibly controlled by computer software(s), commonly used for power supply, signal generation and electrical measurements.

The electrodes are preferably placed so as to set the plane which they determine substantially perpendicular with respect to the average propagation direction of the mass flow in the target volume.

In one preferred embodiment, the electrodes are placed in an annular configuration surrounding the target volume.

As already stated above, the characteristic and preferred features of the apparatus of the present invention are aimed at the purposes and provide the advantages described above concerning the method of the present invention.

In addition to the method and apparatus aspects, the principles of the present invention can be also implemented as a computer program. The computer program according to this aspect of the present invention comprises program code arranged to perform, when run in a suitable data processor, the steps of a method according to the present invention.

In addition to the impedance tomography, the basic principle of the present invention of utilising a state space model comprising the time-dependent flow field of the mass flow within the target volume, thus enabling three-dimensional analysis based on only two-dimensional measurement data, can be applied also in mass flow analysis performed by means of electrical capacitance tomography ECT. In ECT, dielectric permittivity distribution is the electrical property to be determined instead of conductivity.

A typical setup of an ECT measurement system consists of a ring of metal electrodes around a pipe or vessel (or inside a pipe or vessel on the surface of a probe, for example), on either the exterior or interior wall of the pipe/vessel. In most of the cases the electrodes are not in contact with the flowing material but there is a thin layer of insulating material between the electrode and the target volume. The aim of ECT is to visualize the distribution of dielectric (non-conductive) materials by means of contrasts in permittivity.

In ECT, the entire measuring sensor is usually enclosed by a metallic screen to shield off the electromagnetic fields. In a standard measurement procedure an excitation voltage is applied on one of the electrodes (source) while the remaining electrodes (detectors) are grounded, and the charge on each of the detector electrodes is measured. This gives one set of source/detector capacitance measurements. This process continues until each electrode in the sensor system has served once as a source electrode, thus completing the collection of all mutual capacitance measurements between all electrode pairs.

When the approach of the present invention is applied in the case of ECT, a small modification to the forward model compared to EIT needs to be made but the basic idea of the 3D analysis based on one plane measurement arrangement remains the same. Therefore, the same state space approach with proper flow model and correct forward model can also be utilized in the case of ECT.

DETAILED DESCRIPTION OF THE INVENTION

Preferred exemplary embodiments of the present invention are now described in more detail by means of the following explanation i) about simulations carried out to test the applicability of the single electrode layer measurements for three dimensional target volume imaging, and ii) about an apparatus according to the present invention. The explanations are illustrated by the accompanying figures wherein

Figure 1 shows the target volume geometry and the electrode configuration used in the simulations,
Figures 2 - 4 show the results achieved in the simulations, and
Figure 5 shows a schematic figure of an exemplary apparatus according to the present invention.

### i) Simulations

The observation model used in the simulations was of the form

$$V_\tau = f_\tau(\theta_\tau) + \varepsilon_\tau \qquad (1)$$

where $V_\tau$ are the measured voltages and the subscript $\tau$ is a discrete time index referring to the time instant ($t = t_\tau$) of the measurement, $f_\tau(\theta_\tau)$ are the corresponding computed voltages, vector $\theta_\tau$ contains all unknown terms present in the evolution and observation models including the conductivity $\sigma_\tau$ and the contact impedance $z_\tau$, and $\varepsilon_\tau$, is the noise. The evolution model of the augmented state variable $\theta_\tau$ is

$$\theta_{\tau+1} = F\theta_\tau + v_\tau, \qquad (2)$$

where the evolution matrix $F$ is obtained by combining all separate evolution models. The state noise related to the augmented model is denoted with $v_\tau$.

The state estimation problem is to find estimates for the unknown state variables $\theta_\tau$, $\tau = 1, 2, ...$ given the observation and evolution models (eqs (1) and (2)) and observations $\{V_k, k \in I\}$ where $I$ is a set of time indices of observations that are available. The state estimation approach leads to a recursive computational algorithm, examples of which are listed above in the summary section. In the simulations at issue, an iterated extended Kalman filter (IEKF) and a fixed interval smoother (FIS) were used.

In the IEKF, nonlinear and non-Gaussian models are replaced by linear and Gaussian approximations and, in addition, it includes an internal iteration to find an optimal linearization point $\theta^*$. Given the initial point $\theta_{1|0}$ and covariance $\Gamma_{1|0}$ and a guess for the linearization point $\theta^*$, the IEKF equations related to the above state space model are for i = 1 $: n$

$$G = \Gamma_{\tau|\tau-1} J_\tau(\theta^*)^T (J_\tau(\theta^*)\Gamma_{\tau|\tau-1} J_\tau(\theta^*)^T + \Gamma_{\varepsilon\tau})^{-1} \qquad (3)$$

$$\theta^* = \theta_{\tau|\tau-1} + G_\tau(V_\tau - (V(\theta^*) + J_\tau(\theta^*)(\theta_{\tau|\tau-1} - \theta^*)) \qquad (4)$$

and

$$G_\tau = \Gamma_{\tau|\tau-1} J_\tau(\theta^*)^T (J_\tau(\theta^*)\Gamma_{\tau|\tau-1} J_\tau(\theta^*)^T + \Gamma_{\varepsilon\tau})^{-1} \qquad (5)$$

$$\theta_{\tau|\tau} = \theta_{\tau|\tau-1} + G_\tau(V_\tau - (V(\theta^*) + J_\tau(\theta^*)(\theta_{\tau|\tau-1} - \theta^*)) \qquad (6)$$

$$\Gamma_{\tau|\tau} = (I - GJ_\tau(\theta^*))\Gamma_{\tau|\tau-1} \qquad (7)$$

$$\theta_{\tau+1|\tau} = F\theta_{\tau|\tau} \qquad (8)$$

$$\Gamma_{\tau+1|\tau} = F\Gamma_{\tau|\tau}F^T + \Gamma_{v_\tau} \qquad (9)$$

where $J_\tau(\theta) = \dfrac{\partial f_\tau(\theta)}{\partial \theta}$ and $n$ is the number of internal iterations.

If the linearization point $\theta^*$ is fixed, the internal iteration, i.e. the loop of equations (3) and (4), vanishes and we result in the Kalman filter equations.

The fixed interval smoother (FIS) estimates $\theta_{\tau|\tau_{max}}$ and the associated covariances can be obtained from the IEKF results with the backward recursion

$$\Xi_{\tau-1} \;=\; \Gamma_{\tau-1|\tau-1} F^T \Gamma_{\tau|\tau-1}^{-1} \hspace{4cm} (10)$$

$$\theta_{\tau-1|\tau_{max}} \;=\; \theta_{\tau-1|\tau-1} \;+\; \Xi_{\tau-1}(\theta_{\tau|\tau_{max}} - \theta_{\tau|\tau-1}) \hspace{2cm} (11)$$

$$\Gamma_{\tau|\tau_{max}} \;=\; \Gamma_{\tau-1|\tau-1} \;+\; \Xi_{\tau-1}(\Gamma_{\tau|\tau_{max}} - \Gamma_{\tau|\tau-1})\Xi_{\tau-1}^{T}\,. \hspace{1.5cm} (12)$$

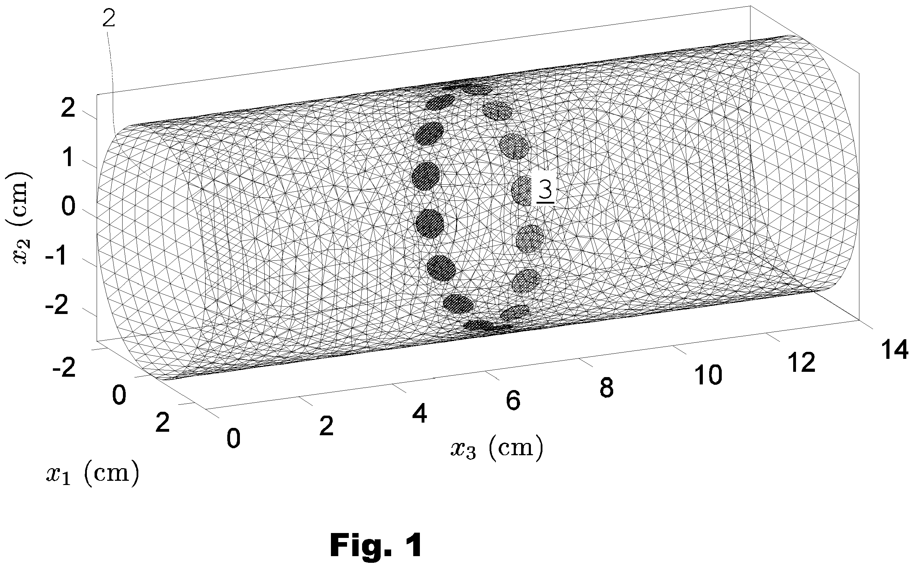

The geometry used in the simulations comprised a straight circular pipe with a diameter of 4.8 cm. As shown in Figure 1, the length of the target volume 2 was 14 cm. The electrodes 3 were located in the middle of the volume and arranged as an annular electrode ring surrounding the inner volume of the pipe, i.e. the target volume 2. Figure 1 shows that the electrode ring lies in a plane perpendicular with respect to the longitudinal direction of the pipe, which direction in this case coincides with the average direction of the mass flow.

The velocity profile of the mass flow was "turbulent-like" with maximum flow speed of 75 $cm\,s^{-1}$. The background conductivity of the material flowing in the pipe was time-varying and, in addition, there were also small resistive non-diffusing objects drifting with the flow. The background conductivity was generated using a FEM simulation in a cylindrical mesh so that we specified a spatially homogeneous and temporally smoothly varying conductivity distribution at the input flow boundary. The background conductivity varied in the range of 0.29 - 0.57 $\Omega^{-1}cm^{-1}$.

The non-diffusing objects were added to the background conductivity simply by creating ellipsoids of varying dimensions and cross-sectional positions and by specifying a conductivity distribution within the ellipsoid. The ellipsoidal objects were added sequentially one at a time in the target volume. Appropriate regions of the background conductivity were then replaced by these ellipsoidal conductivity distributions, and the rate of change in their position was specified by the flow velocity in the central point of the ellipsoid.

Noiseless EIT observations were generated with the FEM simulation assuming that the measurements can be obtained instantaneously. The contact impedances were assumed to depend on the average conductivity in domain $\Omega$, and all electrodes had an equal contact impedance. The number of electrodes was $N_{el}$ =16 and a cycle of eight different opposite 2 mA current injections was used repeatedly. Voltages were measured between adjacent electrodes and the number of measurements at each time instant was $N_{meas}$ =16. The time between subsequent observations was 10 milliseconds. The FEM approximations of the complete electrode model and the convection-diffusion model were implemented in a dense mesh that is visualized in Figure 1. The state estimation problem was solved in a different, smaller, mesh to avoid committing inverse crimes.

Gaussian noise $\varepsilon_\tau \sim N(0, \Gamma_{\varepsilon_\tau})$ was added to noiseless measurement data in order to simulate errors resulting from the measurement electronics and the environment. The measurement noise covariance was of the form $\Gamma_{\varepsilon_\tau} = \delta^2 I$, where $\delta$=0.005.

The contact impedances of the electrodes were estimated separately, since they all were employed for current injections.

For the initialization of the IEFK, the "best homogeneous estimate" $\theta_{bh} = [\sigma_{bh}\ z_{bh}]^T$ was computed, i.e. the least squares estimate when both the conductivity distribution and the contact impedances are described with single parameters. The IEKF was initialized by setting all contact impedances in the augmented state vector $\theta_{1|0}$ to $z_{bh}$ and all conductivities to $\sigma_{bh}$ while the terms representing the inhomogeneous part were set to zero.

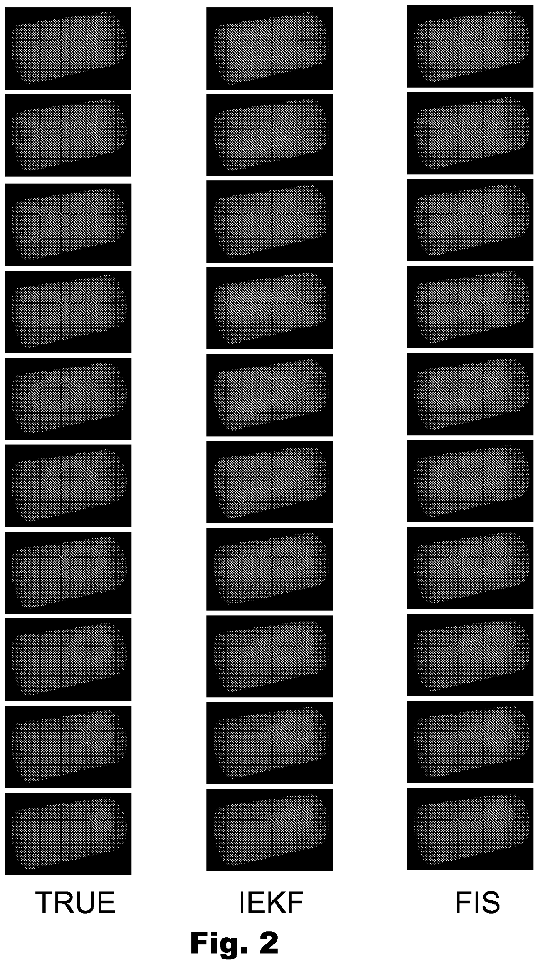

The IEKF and FIS estimates were computed with the recursions described above. A sequence of estimates as well as the true conductivity distribution for one ellipsoidal object drifting through the target volume is shown in Figure 2. The left column in the figure shows the actual ("true") conductivity distribution, the middle column the IEKF estimates, and the right column the FIS estimates. It can be seen that the ellipsoidal object cannot be reconstructed with the IEKF until the object has reached the electrode layer, while the object can be seen in every FIS estimate. This difference results from the different data sets used in the IEKF and the FIS, as explained in the following.

Concerning the IEKF estimates, since EIT measurements are sensitive to the conductivity distribution in the close neighbourhood of the electrode plane, the variations of the conductivity taking place elsewhere cannot be seen by the measurements. Thus, the first indications of the objects are obtained when the object has reached the electrode layer. When passing the electrode plane, the flowing material is "scanned" by the EIT measurements that provide information on the conductivity distribution in the neighbourhood of the electrode layer.

In the FIS, an estimate for each time instant is computed using also data related to one or more later time instants. This causes a delay between the observations and the estimates but it also improves the quality of the estimates. Especially on the upstream side of the electrode layer, the estimates are significantly more accurate than the IEKF estimates.

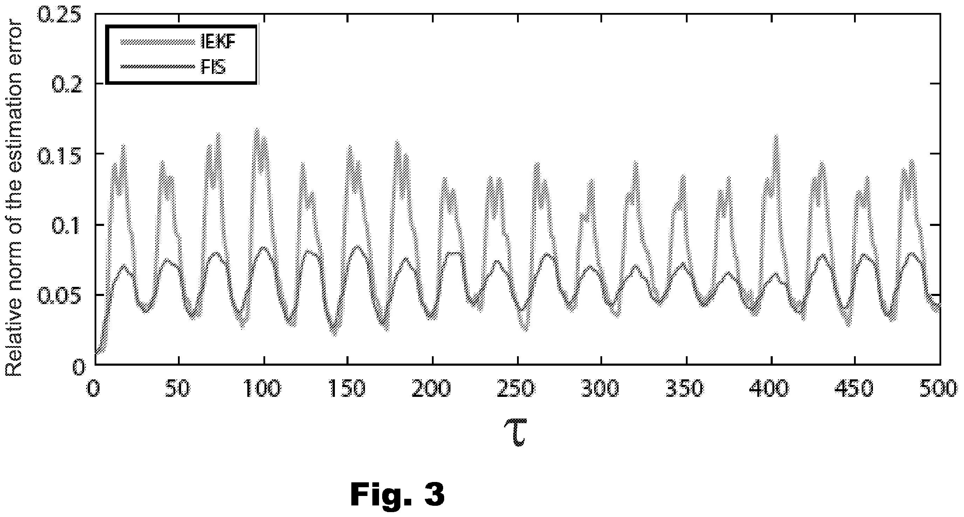

In order to quantify the quality of the estimates for the conductivity, the relative norms of estimation errors at each time instant were computed, and they are shown in Figure 3. In the curves of Figure 3, each peak denotes one ellipsoidal object drifting through the pipe segment forming the target volume. In other words, in both estimates the relative error norms are smallest at those time instants when the ellipsoidal objects are not in the pipe segment. Then also the accuracies of the IEKF and FIS estimates are close to each other with typical values of 3-4 %. When the objects are present within the investigated pipe segment, the maximum relative error norm of the IEKF and FIS estimates are about 13-15 % and 6-7 %, respectively.

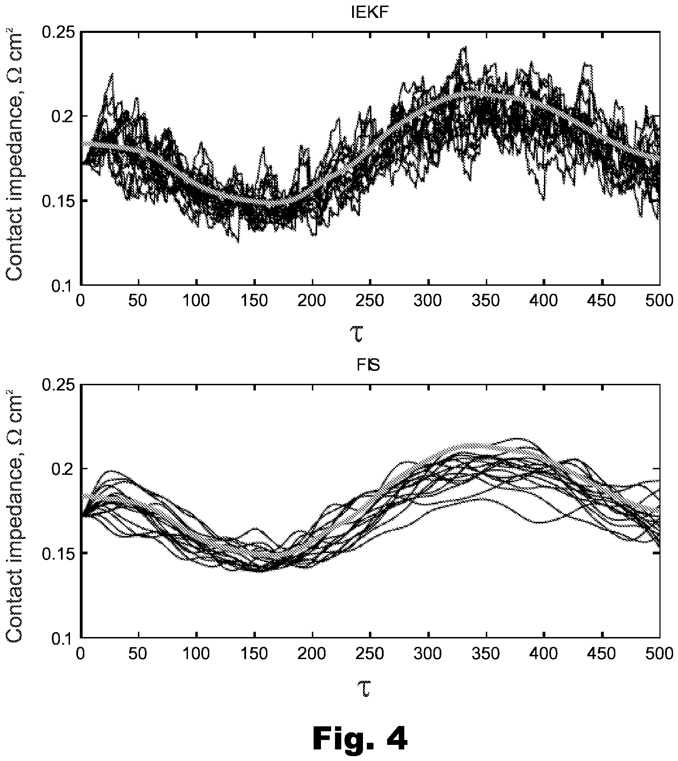

In addition to the conductivity distribution, also the contact impedances were to be estimated. In data generation, the contact impedances of all electrodes were identical. However, in data processing, the electrodes were described with separate values. The true contact impedance and the estimated values are shown in the graphs of Figure 4. The upper graph shows the IEKF estimates and the lower one the FIS estimates. The estimates follow the actual value represented by the bold line, but temporal changes are rather rapid especially in the IEKF estimates.

The simulation results shown in the Figures 2 - 4 and discussed above clearly prove the feasibility of the present invention in three dimensional mass flow imaging. As discussed above, in the IEKF estimates the estimation errors can be rather large on the upstream side of the electrode layer since the observations do not carry information from that region. The observations update the estimates in the region of the electrode layer and from that on, i.e. on the downstream side of the electrode layer, the quality of the estimates depends on the accuracy of the evolution model. The problem with the accuracy in the upstream region can be tackled with smoother algorithms in which also data from the later time instants is used in the estimation of the state of the system at some specific time instant.

ii) Apparatus

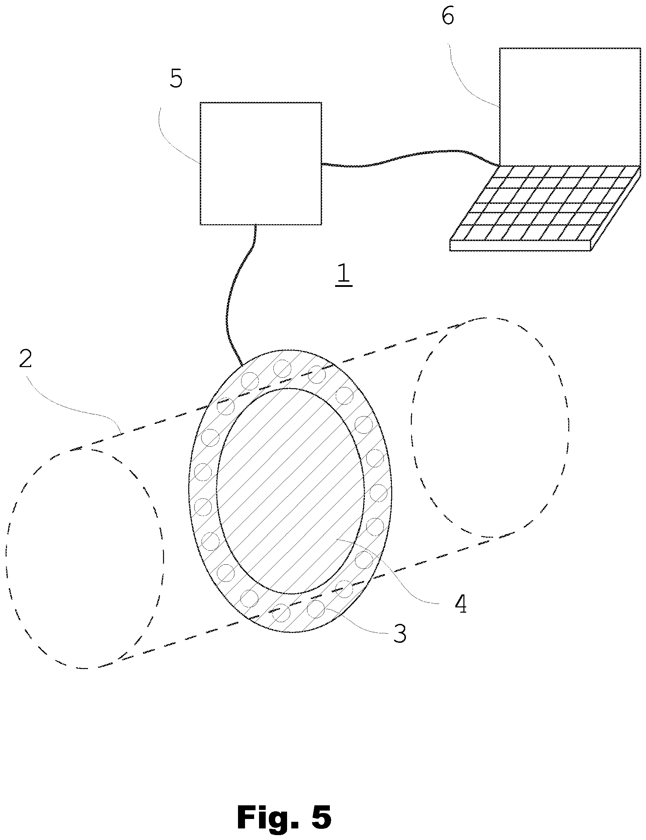

The apparatus of figure 5 comprises electrodes 3 arranged in a ring-like configuration surrounding the target volume 2 of a mass flow. The electrode ring lies in a plane 4 which is perpendicular with respect to the longitudinal direction of the target volume, i.e. the average direction of the mass flow. The electrodes are connected to a signal processing unit 5 comprising electronics needed in generating and supplying to the electrodes the excitation signals as well as measuring the response signals between selected electrodes. Signal generation and measurement as well as couplings between the signal processing unit 5 and the electrodes 3 are controlled by a computer 6 with proper software(s) installed. Also the measurement signal collection and further processing in order to finally form the conductivity distribution within the target volume are performed by means of the computer.

The apparatus of Figure 5 is used and it operates according to the principles of the method described above in this document. For example, the computer 6 with its software(s) together with the signal processing unit 5 form the means for determining a state space model which defines the relationships between the electrical conductivity, the voltage and the current in the target volume and which also defines the evolution of the electrical conductivity as a function of time; means for comparing the voltages and the currents according to the state space model with the supplied and the measured ones; and modifying means for modifying as needed the state space model to decrease the differences between the calculated and the measured results.

Notification

As is clear for a person skilled in the art, the present invention is not limited to the examples explained above. Instead, the embodiments of the present invention can naturally vary freely within the scope of the claims. Particularly, any principles and practises known in the field can be utilized in the details of the state space model as well as the actual calculation methods.

**Claims**

1. A method for determining the three dimensional conductility distribution of a mass flow in a three dimensional target volume (2), the method comprising the steps of

   - collecting current or voltage values generated by supplying alternating voltage or alternating current to the target volume and measuring the current or the voltage, correspondingly, thereby induced in the target volume;
   - providing a state space model which defines the relationships between the electrical conductivity, the voltage, and the current in the target volume (2), and the evolution of the electrical conductivity as a function of time;
   - comparing the currents and/or the voltages according to the state space model with the supplied and the measured ones;

- modifying as needed the state space model to decrease the differences between the calculated and the measured results until a predetermined consistency is achieved; and
- determining the three dimensional conductivity distribution of the mass flow in the target volume according to the modified state space model, **characterised in that**
- the collected current or voltage values are selected to consist of results of measurements performed substantially within one plane (4); and
- the state space model is provided so as to comprise the time-dependent flow field of the mass flow within the target volume (2).

2. A method according to claim 1, **characterised in that** the evolution of the electrical conductivity as a function of time is determined in the state space model by means of a convection-diffusion model.

3. A method according to claim 1 or 2, **characterised in that** for the steps of comparing the currents and/or voltages according to the state space model with the supplied and the measured ones and modifying as needled the state space model, a data set comprising current and/or voltage values according to the state space model, correspondingly, within the target volume (2) is generated.

4. A method according to any of claims 1 to 3, **characterised in that** the step of collecting current or voltage values comprises, using a plurality of electrodes (3) in a measuring connection with the target volume, the electrodes being placed substantially within one plane, supplying alternating voltage or alternating current to the target volume between two of the electrodes (3), and measuring the current or the voltage, correspondingly, between two of the electrodes (3).

5. A method according to claim 4, **characterised in that** the electrodes (3) are placed so as to set the plane (4) which they determine substantially perpendicular with respect to the average propagation direction of the mass flow in the target volume (2).

6. A method according to claim 4 or 5, **characterised in that** the electrodes (3) are placed in an annular configuration surrounding the target volume (2).

7. An apparatus (1) for determining the three dimensional conductivity distribution of a mass flow in a three dimensional target volume (2), the apparatus comprising

- collecting means (3, 5, 6) for collecting current or voltage values generated by supplying alternating voltage or alternating current to the target volume and measuring the current or the voltage, correspondingly, thereby induced in the target volume;
- first determining means (5, 6) for determining a state space model which defines the relationships between the electrical conductivity, the voltage, and the current in the target volume (2), and the evolution of the electrical conductivity as a function of time;
- comparing means (5, 6) for comparing the currents and/or the voltages according to the state space model with the supplied and the measured ones;
- modifying means (5, 6) for modifying as needed the state space model to decrease the differences between the calculated and the measured results; and
- second determining (5, 6) means for determining the three dimensional conductivity distribution of the mass flow in the target volume according to the modified state space model,

**characterised in that**

- the collecting means are arranged to select the collected current or voltage values so as to consist of results of measurements performed substantially within one plane (4); and
- the first determining means (5, 6) are arranged to determine the state space model so as to comprises the time-dependent flow field of the mass flow within the target volume (2).

8. An apparatus (1) according to claim 7, **characterised in that** the first determining means (5, 6) are arranged to determine the evolution of the electrical conductivity as a function of time in the state space model by means of a convection-diffusion model.

9. An apparatus (1) according to claim 7 or 8, **characterised in that** for comparing the currents and/or voltages according to the state space model with the supplied and the measured ones and modifying as needed the state space model, the apparatus comprises means for generating a data set comprising current and/or voltage values according to the state space model, correspondingly, within the target volume (2) .

10. An apparatus (1) according to any of claims 7 to 9, **characterised in that** the collecting means comprise electrodes (3) in a measuring connection with the target volume, the electrodes being placed substantially within one plane, and supplying and measuring means (5, 6) for supplying alternating voltage or alternating current to the target volume between two of the electrodes (3) and measuring the current or the voltage between two of the electrodes (3).

11. An apparatus (1) according to claim 10, **characterised in that** the electrodes (3) are placed so as to set the plane (4) which they determine substantially perpendicular with respect to the average propagation direction of the mass flow in the target volume (2).

12. An apparatus (1) according to claim 10 or 11, **characterised in that** the electrodes (3) are placed in an annular configuration surrounding the target volume (2).

13. A computer program comprising program code arranged to perform, when run in a data processor, the method steps according to any of claims 1 to 6.

**Patentansprüche**

1. Ein Verfahren zur Bestimmung der dreidimensionalen Leitfähigkeitsverteilung eines Masseflusses in einem dreidimensionalen Zielvolumen (2), das Verfahren die folgenden Schritte umfassend

 - Sammeln von Strom- oder Spannungswerten, die durch Anlegen von Wechselspannung oder Wechselstrom an das Zielvolumen erzeugt werden, und Messen des Stromes oder der Spannung, die dadurch entsprechend im Zielvolumen erzeugt wird;
 - Bereitstellen eines Zustandsraummodelles, welches die Beziehungen zwischen der elektrischen Leitfähigkeit, der Spannung und dem Strom im Zielvolumen (2), und der Entwicklung der elektrischen Leitfähigkeit als eine Funktion der Zeit definiert;
 - Vergleichen der Ströme und/oder der Spannungen entsprechend des Zustandsraummodelles mit den angelegten und den gemessenen Strömen und/oder Spannungen;
 - Gegebenenfalls modifizieren des Zustandsraummodelles, um die Unterschiede zwischen den berechneten und den gemessenen Ergebnissen zu reduzieren, bis eine festgelegte Konsistenz erreicht wird; und
 - Bestimmen der dreidimensionalen Leitfähigkeitsverteilung des Masseflusses im Zielvolumen entsprechend des modifizierten Zustandsraummodelles,

 **dadurch gekennzeichnet, dass**

 - Die gesammelten Strom- oder Spannungswerte ausgewählt sind, um aus Ergebnissen der Messungen, die im Wesentlichen in einer Ebene (4) durchgeführt werden, zu bestehen; und
 - Das Zustandsraummodell bereitgestellt wird, um das zeitabhängige Flussfeld des Masseflusses innerhalb des Zielvolumens (2) zu umfassen.

2. Ein Verfahren nach Anspruch 1, **dadurch gekennzeichnet, dass** die Entwicklung der elektrischen Leitfähigkeit als eine Funktion der Zeit im Zustandsraummodell mittels eines Konvektions-Diffusions-Modells bestimmt wird.

3. Ein Verfahren nach Anspruch 1 oder 2, **dadurch gekennzeichnet, dass** für die Schritte des Vergleichens der Ströme und/oder Spannungen entsprechend des Zustandsraummodells mit den angelegten und den gemessenen Strömen und/oder Spannungen und gegebenenfalls des Modifizierens des Zustandsraummodells, ein Datenset, die Strom- und/oder Spannungswerte umfassend, nach dem Zustandsraummodell entsprechend innerhalb des Zielvolumens (2) erzeugt wird.

4. Ein Verfahren nach einem der Ansprüche 1 bis 3, **dadurch gekennzeichnet, dass** der Schritt des Sammelns von Strom- oder Spannungswerten mittels einer Mehrzahl an Elektroden (3) in einer Messverbindung mit dem Zielvolumen, wobei die Elektroden im Wesentlichen in einer Ebene angeordnet sind, das Anlegen von Wechselspannung oder Wechselstrom an das Zielvolumen zwischen zwei der Elektroden (3), und das Messen des entsprechenden Stroms oder der Spannung zwischen zwei der Elektroden (3), umfasst.

**5.** Ein Verfahren nach Anspruch 4, **dadurch gekennzeichnet, dass** die Elektroden (3) so angeordnet sind, um eine Ebene (4) zu bestimmen, welche sie im Wesentlichen senkrecht in Bezug auf die durchschnittliche Ausbreitungsrichtung des Masseflusses in dem Zielvolumen (2) festlegen.

**6.** Ein Verfahren nach Anspruch 4 oder 5, **dadurch gekennzeichnet, dass** die Elektroden (3) in einer ringförmigen Anordnung um das Zielvolumen (2) angeordnet sind.

**7.** Eine Vorrichtung (1) zum Bestimmen der dreidimensionalen Leitfähigkeitsverteilung eines Masseflusses in einem dreidimensionalen Zielvolumen (2), die Vorrichtung umfassend

- Sammelmittel (3, 5, 6) zum Sammeln von Strom- oder Spannungswerten, die durch Anlegen von Wechselspannung oder Wechselstrom an das Zielvolumen erzeugt werden, und Messen des Stromes oder der Spannung, die dadurch entsprechend im Zielvolumen erzeugt werden;
- Erste Bestimmungsmittel (5, 6) zum Bestimmen eines Zustandsraummodelles, welches die Beziehungen zwischen der elektrischen Leitfähigkeit, der Spannung und des Stromes in dem Zielvolumen (2), und der Entwicklung der elektrischen Leitfähigkeit als eine Funktion der Zeit definiert;
- Vergleichsmittel (5, 6) zum Vergleichen der Ströme und/oder der Spannungen entsprechend des Zustandsraummodelles mit den angelegten und den gemessenen Strömen und/oder Spannungen;
- Modifizierungsmittel (5, 6), gegebenenfalls zum Modifizieren des Zustandsraummodelles, um die Unterschiede zwischen den berechneten und den gemessenen Ergebnissen zu reduzieren; und
- Zweite Bestimmungsmittel (5, 6) zum Bestimmen der dreidimensionalen Leitfähigkeitsverteilung des Masseflusses im Zielvolumen entsprechend des modifizierten Zustandsraummodelles,

**dadurch gekennzeichnet, dass**

- Die Sammelmittel angeordnet sind, um die gesammelten Strom- oder Spannungswerte auszuwählen, sodass sie aus Ergebnissen der Messungen, die im Wesentlichen in einer Ebene (4) durchgeführt werden, bestehen; und
- Die ersten Bestimmungsmittel (5, 6) angeordnet sind, um das Zustandsraummodell so festzulegen, dass es ein zeitabhängiges Flussfeld des Masseflusses innerhalb des Zielvolumens (2) umfasst.

**8.** Eine Vorrichtung (1) nach Anspruch 7, **dadurch gekennzeichnet, dass** die ersten Bestimmungsmittel (5, 6) so angeordnet sind, dass sie die Entwicklung der elektrischen Leitfähigkeit als eine Funktion der Zeit in dem Zustandsraummodell mittels eines Konvektions-Diffusions-Modells bestimmen.

**9.** Eine Vorrichtung (1) nach Anspruch 7 oder 8, **dadurch gekennzeichnet, dass** für das Vergleichen der Ströme und/oder Spannungen nach dem Zustandsraummodell mit den angelegten und den gemessenen Strömen und/oder Spannungen und gegebenenfalls Modifizieren des Zustandsraummodells, die Vorrichtung Mittel zum Erzeugen eines Datensets, Strom- und/oder Spannungswerte nach dem Zustandsraummodell umfassend, entsprechend innerhalb des Zielvolumens (2) umfasst.

**10.** Eine Vorrichtung (1) nach einem der Ansprüche 7 bis 9, **dadurch gekennzeichnet, dass** die Sammelmittel Elektroden (3) in einer Messverbindung mit dem Zielvolumen, wobei die Elektroden im Wesentlichen in einer Ebene angeordnet sind, und Anlege- und Messmittel (5, 6), zum Anlegen von Wechselspannung oder Wechselstrom an das Zielvolumen zwischen zwei der Elektroden (3) und zum Messen des Stroms oder der Spannung zwischen zwei der Elektroden (3), umfassen.

**11.** Eine Vorrichtung (1) nach Anspruch 10, **dadurch gekennzeichnet, dass** die Elektroden (3) so angeordnet sind, um eine Ebene (4) zu bestimmen, welche sie im Wesentlichen senkrecht in Bezug auf die durchschnittliche Ausbreitungsrichtung des Masseflusses in dem Zielvolumen (2) festlegen.

**12.** Eine Vorrichtung (1) nach Anspruch 10 oder 11, **dadurch gekennzeichnet, dass** die Elektroden (3) in einer ringförmigen Anordnung um das Zielvolumen (2) angeordnet sind.

**13.** Ein Computerprogramm umfassend eines Programmcodes, der eingerichtet ist, sofern er in einem Datenprozessor läuft, die Verfahrensschritte nach einem der Ansprüche 1 bis 6 durchzuführen.

**Revendications**

1. Procédé de détermination de la distribution de conductivité tridimensionnelle d'un flux massique dans un volume cible tridimensionnel (2), le procédé comprenant les étapes consistant à :

   - recueillir des valeurs de courant ou de tension générées en fournissant une tension alternative ou un courant alternatif au volume cible et mesurer le courant ou la tension, de manière correspondante, induit(e) dans le volume cible ;
   - fournir un modèle spatial d'état qui définit la relation entre la conductivité électrique, la tension et le courant dans le volume cible (2) et l'évolution de la conductivité électrique en fonction du temps ;
   - comparer les courants et/ou les tensions en fonction du modèle spatial d'état à ceux fournis et à ceux mesurés ;
   - modifier selon les besoins le modèle spatial d'état pour réduire les différences entre les résultats calculés et ceux mesurés jusqu'à ce qu'une consistance prédéterminée soit obtenue ; et
   - déterminer la distribution de conductivité tridimensionnelle du flux massique dans le volume cible selon le modèle spatial d'état modifié,

   **caractérisé en ce que** :

   - les valeurs de courant ou de tension recueillies sont sélectionnées pour constituer les résultats de mesures effectuées sensiblement dans un plan (4) ; et
   - le modèle spatial d'état est fourni de manière à comprendre le champ de flux fonction du temps du flux massique dans le volume cible (2).

2. Procédé selon la revendication 1, **caractérisé en ce que** l'évolution de la conductivité électrique en fonction du temps est déterminée dans le modèle spatial d'état au moyen d'un modèle de convexion-diffusion.

3. Procédé selon la revendication 1 ou 2, **caractérisé en ce que**, pour les étapes de comparaison des courants et/ou des tensions selon le modèle spatial d'état avec ceux fournis et ceux mesurés et de modification selon les besoins du modèle spatial d'état, un jeu de données comprenant des valeurs de courant et/ou de tension selon le modèle spatial d'état, de manière correspondante, dans le volume cible (2) est généré.

4. Procédé selon l'une quelconque des revendications 1 à 3, **caractérisé en ce que** l'étape de recueil de valeurs de courant ou de tension comprend l'utilisation d'une pluralité d'électrodes (3) dans une connexion de mesure avec le volume cible, les électrodes étant placées sensiblement dans un plan, la fourniture d'une tension alternative ou d'un courant alternatif au volume cible entre deux des électrodes (3) et la mesure du courant ou de la tension, de manière correspondante, entre deux des électrodes (3).

5. Procédé selon la revendication 4, **caractérisé en ce que** les électrodes (3) sont placées de manière à régler le plan (4) qu'elles déterminent sensiblement perpendiculairement par rapport à la direction de propagation moyenne du flux massique dans le volume cible (2).

6. Procédé selon la revendication 4 ou 5, **caractérisé en ce que** les électrodes (3) sont placées dans une configuration annulaire entourant le volume cible (2).

7. Appareil (1) pour déterminer la distribution de conductivité tridimensionnelle d'un flux massique dans un volume cible tridimensionnel (2), l'appareil comprenant :

   - des moyens collecteurs (3, 5, 6) pour recueillir des valeurs de courant ou de tension générées en fournissant une tension alternative ou un courant alternatif au volume cible et en mesurant le courant ou la tension, de manière correspondante, ainsi induit(e) dans le volume cible ;
   - des premiers moyens de détermination (5, 6) pour déterminer un modèle spatial d'état qui définit la relation entre la conductivité électrique, la tension et le courant dans le volume cible (2) et l'évolution de la conductivité électrique en fonction du temps,
   - des moyens de comparaison (5, 6) pour comparer les courants et/ou les tensions selon le modèle spatial d'état avec ceux fournis et ceux mesurés ;
   - des moyens de modification (5, 6) pour modifier selon les besoins le modèle spatial d'état afin de réduire les différences entre les résultats calculés et les résultats mesurés ; et
   - des seconds moyens de détermination (5, 6) pour déterminer la distribution de conductivité tridimensionnelle du flux massique dans le volume cible selon le modèle spatial d'état modifié,

**caractérisé en ce que** :

- les moyens collecteurs sont agencés pour sélectionner les valeurs de courant ou de tension recueillies de manière qu'elles soient constituées de résultats de mesures effectuées sensiblement dans un plan (4) ; et
- les premiers moyens de détermination (5, 6) sont agencés pour déterminer le modèle spatial d'état de manière à comprendre le champ de flux fonction du temps du flux massique dans le volume cible (2).

8. Appareil (1) selon la revendication 7, **caractérisé en ce que** les premiers moyens de détermination (5, 6) sont agencés pour déterminer l'évolution de la conductivité électrique en fonction du temps dans le modèle spatial d'état au moyen d'un modèle de convection-diffusion.

9. Appareil (1) selon la revendication 7 ou 8, **caractérisé en ce que**, pour comparer les courants et/ou les tensions selon le modèle spatial d'état à ceux fournis et ceux mesurés et modifier selon les besoins le modèle spatial d'état, l'appareil comprend des moyens pour générer un jeu de données comprenant des valeurs de courant et/ou de tension selon le modèle spatial d'état, de manière correspondante, dans le volume cible (2).

10. Appareil (1) selon l'une quelconque des revendications 7 à 9, **caractérisé en ce que** les moyens collecteurs comprennent des électrodes (3) dans une connexion de mesure avec le volume cible, les électrodes étant placées sensiblement dans un plan, et des moyens de fourniture et de mesure (5, 6) pour fournir une tension alternative ou un courant alternatif au volume cible entre deux des électrodes (3) et mesurer le courant ou la tension entre deux des électrodes (3).

11. Appareil (1) selon la revendication 10, **caractérisé en ce que** les électrodes (3) sont placées de manière à régler le plan (4) qu'elles déterminent sensiblement perpendiculairement par rapport à la direction de propagation moyenne du flux massique dans le volume cible (2).

12. Appareil (1) selon la revendication 10 ou 11, **caractérisé en ce que** les électrodes (3) sont placées dans une configuration annulaire entourant le volume cible (2).

13. Programme informatique comprenant un code de programme agencé pour effectuer, lorsqu'il tourne dans un processeur de données, les étapes de procédé selon l'une quelconque des revendications 1 à 6.

**Fig. 1**

EP 2 483 672 B1

TRUE                    IEKF                    FIS

**Fig. 2**

**Fig. 3**

**Fig. 4**

**Fig. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. SEPPÄNEN ; M. VAUHKONEN ; P.J. VAUHKONEN ; E. SOMERSALO ; J.P. KAIPIO.** State estimation with fluid dynamical evolution models in process tomography -- An application with impedance tomography. *Inverse Problems,* 2001, vol. 17, 467-483
- **A. SEPPÄNEN ; M. VAUHKONEN ; P.J. VAUHKONEN ; E. SOMERSALO ; J.P. KAIPIO.** Fluid dynamical models and state estimation in process tomography: Effect due to inaccuracies in flow fields. *J. Electr. Imag.,* 2001, vol. 10 (3), 630-640
- **A. SEPPÄNEN ; L. HEIKKINEN ; T. SAVOLAINEN ; A. VOUTILAINEN ; E. SOMERSALO ; J.P. KAIPIO.** An experimental evaluation of state estimation with fluid dynamical models in process tomography. *Chemical Engineering Journal,* 2007, vol. 127, 23-30
- **A. SEPPÄNEN ; M. VAUHKONEN ; P.J. VAUHKONEN ; A. VOUTILAINEN ; J.P. KAIPIO.** State estimation in three dimensional impedance imaging - Use of fluid dynamical evolution models. *International Journal for Numerical Methods in Engineering,* 2008, vol. 73, 1651-1670